# EUROPEAN PATENT SPECIFICATION

(11) **EP 1 139 975 B1**
(45) Date of publication and mention of the grant of the patent: **18.06.2003**
(21) Application number: 98964200.4
(22) Date of filing: 22.12.1998
(51) Int. Cl.: A61K 7/00

(54) **TRANSPARENT SKIN CARE COMPOSITIONS**
TRANSPARENTE KÖRPERPFLEGEMITTEL
COMPOSITIONS TRANSPARENTES DE SOINS POUR LA PEAU

(43) Date of publication of application: 10.10.2001
(73) Proprietor: THE PROCTER & GAMBLE COMPANY, Cincinnati, Ohio 45202 (US)
(72) Inventor: TANAKA, Hidekazu, Yokaichi-shi Shiga 527-0086 (JP); MORI, Kiyoaki, Yasu-gun Shiga 520-2351 (JP); TSUNETSUGU, Shuichi, Kobe 658-0000 (JP)
(74) Representative: McKinney, Victoria
(86) International application number: US9827228
(87) International publication number: WO00037029

(56) References cited:
- EP-A- 0 349 150
- EP-A- 0 487 958
- US-A- 5 474 776

## Description

### FIELD

The present invention relates to a transparent skin care composition. In particular, it relates to a transparent skin care composition for moisturizing the skin.

### BACKGROUND

A wide variety of skin care compositions are topically applied to achieve cosmetic and/or medical benefits such as moisturizing, wrinkle prevention, protection of skin aging, and environmental damage treatment. Exemplary types of such compositions include skin conditioning compositions, sunscreen compositions, and skin renewal compositions.

Oil-in-water and/or water-in-oil emulsions are well-known product forms in the field of *e.g.*, cosmetic and/or pharmaceutical compositions, in particular cosmetic products such as lotions, tonics, serums or toilet waters including transparent and semi-transparent types. Generally, such emulsions contain oil, water, and surfactants, which are particularly useful for emulsifying the oil and water to provide uniform mixture. Oils and various moisture retaining type agents, which are used as emolients, can provide a pleasant feel to the skin. The higher the concentration of oil, the more the moisturization effect to the skin.

However, increasing the oil concentration in the composition, particularly in a transparent composition, tends to require sufficient levels of surfactants for emulsifying the oil compound to obtain a transparent composition that is uniformly mixed as described in EP0 487 958 B1. Such concentration may cause separation of oil and water contents, resulting in short-lived product stability. In addition, some oils, *e*.*g*., oils having high molecular weights or highly-substituted structures, tend to be difficult to solubilize with surfactants that are commonly used in cosmetics.

Based on the foregoing, there is a need for a transparent skin care composition providing skin moisturizing efficacy without compromising product stability or consumer acceptance. None of the existing art provides all of the advantages and benefits of the present invention.

### SUMMARY

The present invention is directed to a transparent skin care composition comprising:
(a) a surfactant combination comprising i), ii) and iii):
   i) polyoxyethylene sorbit tetralkyl ester;
   ii) polyoxyethylene castor oil ester and/or polyoxyethylene hydrogenated castor oil ester; and
   iii) polyoxyethylene alkyl phosphate or salts
(b) an oil compound
(c) a polyhydric alcohol; and
(d) water.

The oil compound is substantially solubilized in the transparent skin care composition.

These and other features, aspects, and advantages of the present invention will become better understood from a reading of the following description, and appended claims.

### DETAILED DESCRIPTION

While the specification concludes with claims particularly pointing out and distinctly claiming the invention, it is believed that the present invention will be better understood from the following description.

All percentages, ratios, and levels of ingredients referred to herein are based on the actually total amount of the composition, unless otherwise indicated.

All measurements referred to herein are made at 25°C unless otherwise specified.

Herein, "comprising" means that other steps and other ingredients which do not affect the end result can be added. This term encompasses the terms "consisting of" and "consisting essentially of."

Herein, "topical application" means to apply or spread a material onto the surface of the skin.

Herein, "dermatologically-acceptable," means that the compositions or components thereof so described are suitable for use in contact with human skin without undue toxicity, incompatibility, instability, irritation allergic response, and the like, commensurate with a reasonable benefit/risk ratio.

Herein, "cosmetically-acceptable carrier," means one or more compatible dermatologically-acceptable solid or liquid filler diluents or encapsulating substances.

Herein, "safe and effective amount," means an amount of a compound or composition sufficient to significantly induce a positive benefit, preferably a positive skin appearance or feel benefit, including independently the benefits disclosed herein, but low enough to avoid serious side effects, *i*.*e*., to provide a reasonable benefit to risk ratio, within the scope of sound judgment of the skilled artisan.

Herein, "mixtures" is meant to include a simple combination of materials and any compounds that may result from their combination.

All ingredients such as actives and other ingredients useful herein may be categorized or described by their cosmetic and/or therapeutic benefit or their postulated mode of action. However, it is to be understood that the active and other ingredients useful herein can, in some instances, provide more than one cosmetic and/or therapeutic benefit or operate via more than one mode of action. Therefore, classifications herein are made for the sake of convenience and are not intended to limit an ingredient to the particularly stated application or applications listed.

The present invention is directed to a transparent skin care composition comprising two or more surfactants, an oil compound, a polyhydric alcohol, and water. The surfactants are selected from the group consisting of polyoxyethylene sorbit tetraalkyl ester (POE sorbit tetraalkyl ester), polyoxyethylene castor oil ester and/or polyoxyethylene hydrogenated castor oil ester (POE castor oil ester and/or POE hydrogenated castor oil ester), polyoxyethylene alkyl phosphate or salts (POE alkyl phosphate or salts), and mixtures thereof. The oil compound in the composition is substantially solubilized in the transparent skin care composition.

The combination of surfactants can emulsify various types of oils, providing pleasant skin feel, and moisturization of the skin without greasy feel. In addition, stabilized compositions with long shelf life may be provided.

### A. Surfactant

The transparent skin care composition of the present invention includes a surfactant combination as defined in claim 1. Without being bound by the theory, it is believed that the combination of surfactants significantly increases the oil solubilization, especially those oils which tend to difficult to be solubilized/emulsified under conventional ways and conditions. The surfactant is present in the composition from 0.001% to 5.0%, preferably from 0.01% to 2.0%.

The surfactants useful herein are selected from the group consisting of POE sorbit tetraalkyl ester, POE castor oil ester and/or POE hydrogenated castor oil ester, POE alkyl phosphate or salts, and mixtures thereof.

Preferably, the combination of surfactants comprises POE sorbit tetraalkyl ester and POE castor oil ester and/or POE hydrogenated castor oil ester; wherein the ratio is from 4 : 1 to 2 : 3. Another combination of surfactants comprises POE alkyl phosphate or salts and POE castor oil ester and/or POE hydrogenated castor oil ester, wherein the ratio is from 4 : 1 to 2 : 3. Further combination comprises POE sorbit tetraalkyl ester and POE alkyl phosphate or salts; wherein the ratio is from 3 : 2 to 2 : 3.

The combination of the present invention contains up to 80% of the POE sorbit tetraalkyl ester, up to 60% of the POE castor oil ester and/or POE hydrogenated castor oil ester, and up to 60% of POE alkyl phosphate or salts by weight of the total amount of surfactant.

Preferred POE sorbit tetraalkyl esters useful herein include POE-40 sorbit tetraoleate and POE-60 sorbit tetrastearate.

Preferred POE castor oil esters and/or POE hydrogenated castor oil esters useful herein include, POE-20 castor oil ester, POE-20 hydrogenated castor oil ester, POE-40 castor oil ester, POE-40 hydrogenated castor oil ester, POE-60 hydrogenated castor oil ester, and POE-80 hydrogenated castor oil ester.

Preferred POE alkyl phosphate or salts useful herein include, triceteth-5 phosphate or salts, tricapryl ether or salts, and di-C₁₂₋₁₅ Pareth-8 phosphate or salts.

### B. Oil Compound

The transparent skin care composition of the present invention includes an oil compound. The oil compound is used as an emollient. The oil compound is present in the composition from 0.001% to 5.0%, preferably from 0.01% to 2.0%.

A wide variety of suitable oil compounds are known and may be used herein and numerous examples can be found in Sagarin, Cosmetics, Science and Technology, 2nd Edition, Vol. 1, pp. 32-43 (1972). Examples of suitable oil compounds include mineral oil, petrolatum, C₇₋₄₀ straight and branched hydrocarbons, C₁₋₃₀ alcohol esters of C₁₋₃₀ carboxylic acids and of C₂₋₃₀ dicarboxylic acids, vegetable oils and hydrogenated vegetable oils animal fats and oils, and C₄₋₂₀ alkyl ethers of polypropylene glycols, C₁₋₂₀ carboxylic acid esters of polypropylene glycols, and di-C₈₋₃₀ alkyl ethers. Oils which are commonly used as perfume agents such as essential oils and synthetic perfume oils can also be used.

In one embodiment, the oil compound is a liquid polyol carboxylic acid ester having a polyol moiety and at least 4 carboxylic acid moieties ester and has a complete melting point of less than 30°C. The polyol moiety is selected from sugars and sugar alcohols containing from about 4 to about 8 hydroxyl groups, and each carboxylic acid moiety has from about 8 to about 22 carbon atoms.

The polyol ester preferred for use herein is a non-occlusive liquid or liquifiable polyol carboxylic acid ester. These polyol esters are derived from a polyol radical or moiety and one or more carboxylic acid radicals or moieties. In other words, these esters contain a moiety derived from a polyol and one or more moieties derived from a carboxylic acid. These carboxylic acid esters can also be derived from a carboxylic acid. These carboxylic acid esters can also be described as liquid polyol fatty acid esters, because the terms carboxylic acid and fatty acid are often used interchangeably by those skilled in the art.

The preferred liquid polyol polyesters employed in this invention comprise certain polyols, especially sugars or sugar alcohols, esterified with at least four fatty acid groups. Accordingly, the polyol starting material must have at least four esterifiable hydroxyl groups. Examples of preferred polyols are sugars, including monosaccharaides and disaccharides, and sugar alcohols. Examples of monosaccharides containing four hydroxyl groups are xylose and arabinose and the sugar alcohol derived from xylose, which has five hydroxyl groups, i.e., xylitol. The monosaccharide, erythrose, is not suitable in the practice of this invention since it only contains three hydroxyl groups, but the sugar alcohol derived from erythrose, *i*.*e*., erythritol, contains four hydroxyl groups and accordingly can be used. Suitable five hydroxyl group-containing monosaccharides are galactose, fructose, and sorbose. Sugar alcohols containing six -OH groups derived from the hydrolysis products of sucrose, as well as glucose and sorbose, *e*.*g*., sorbitol, are also suitable. Examples of disaccharide polyols which can be used include maltose, lactose, and sucrose, all of which contain eight hydroxyl groups.

Preferred polyols for preparing the polyesters for use in the present invention are selected from the group consisting of erythritol, xylitol, sorbitol, glucose, and sucrose. Sucrose is especially preferred.

The polyol starting material having at least four hydroxyl groups is esterified on at least four of the -OH groups with a fatty acid containing from 8 to 22 carbon atoms. Examples of such fatty acids include caprylic, capric, lauric, myristic, myristoleic, palmitic, palmitoleic, stearic, oleic, ricinoleic, linoleic, linolenic, eleostearic, arachidic, arachidonic, behenic, and erucic acid. The fatty acids can be derived from naturally occurring or synthetic fatty acids; they can be saturated or unsaturated, including positional and geometrical isomers. However, in order to provide liquid polyesters preferred for use herein, at least 50% by weight of the fatty acid incorporated into the polyester molecule should be unsaturated. Oleic and linoleic acids, and mixtures thereof, are especially preferred.

The polyol fatty acid polyesters useful in this invention should contain at least four fatty acid ester groups. It is not necessary that all of the hydroxyl groups of the polyol be esterified with fatty acid, but it is preferable that the polyester contain no more than two unesterified hydroxyl groups. Most preferably, substantially all of the hydroxyl groups of the polyol are esterified with fatty acid, *i*.*e*., the polyol moiety is substantially completely esterified. The fatty acids esterified to the polyol molecule can be the same or mixed, but as noted above, a substantial amount of the unsaturated acid ester groups must be present to provide liquidity. To illustrate the above points, a sucrose fatty triester would not be suitable for use herein because it does not contain the required four fatty acid ester groups. A sucrose tetra-fatty acid ester would be suitable, but is not preferred because it has more than two unesterified hydroxyl groups. A sucrose *hexa-*fatty acid ester would be preferred because it has no more than two unesterified hydroxyl groups. Highly preferred compounds in which all the hydroxyl groups are esterified with fatty acids include the liquid sucrose octa-substituted fatty acid esters.

The following are examples of specific polyol fatty acid polyesters containing at least four fatty acid ester groups suitable for use in the present invention: glucose tetraoleate, the glucose tetraesters of soybean oil fatty acids (unsaturated), the mannose tetraesters of mixed soybean oil fatty acids, the galactose tetraesters of oleic acid, the arabinose tetraesters of linoleic acid, xylose tetralinoleate, galactose pentaoleate, sorbitol tetraoleate, the sorbitol hexaesters of unsaturated soybean oil fatty acids, xylitol pentaoleate, sucrose tetraoleate, sucrose pentaoletate, sucrose hexaoleate, sucrose hepatoleate, sucrose octaoleate, and mixtures thereof.

As noted above, highly preferred polyol fatty acid esters are those wherein the fatty acids contain from 14 to 18 carbon atoms.

The preferred liquid polyol polyesters preferred for use herein have complete melting points below 30°C, preferably below 27.5°C, more preferably below 25°C. Complete melting points reported herein are measured by Differential Scanning Calorimetry (DSC).

The polyol fatty acid polyesters suitable for use herein can be prepared by a variety of methods well known to those skilled in the art. These methods include: transesterification of the polyol with methyl, ethyl or glycerol fatty acid esters using a variety of catalysts; acylation of the polyol with a fatty acid chloride; acylation of the polyol with a fatty acid anhydride; and acylation of the polyol with a fatty acid, per se. See U.S. Patent No. 2,831,854; U.S. Patent No. 4,005,196, to Jandacek, issued January 25, 1977; U.S. Patent No. 4,005,196, to Jandacek, issued January 25, 1977.

### C. Polyhydric Alcohol

The transparent skin care composition of the present invention includes a polyhydric alcohol. It is believed that the polyhydric alcohol used herein performs preventing the gelling formation. Higher amount of water concentration, *e.g.*, more than 60% of water may lead to a separation of oil phase from the uniform mixture during the process of transparent composition. The polyhydric alcohol, especially added into the mixture of surfactants and oil compounds, can employ to mix water into uniformly mixed oil phase.

The polyhydric alcohols useful herein are present in the composition from 0.01% to 10.0%, preferably from 0.5% to 2.0%.

Preferred polyhydric alcohols useful herein include, but are not limited to, polyalkylene glycols, more preferably alkylene polyols and their derivatives including glycerin, propylene glycol, dipropylene glycol, tripropylene glycol, polyethylene glycol and derivatives thereof, sorbitol, hydroxypropyl sorbitol, erythritol, threitol, pentaerythritol, xylitol, glucitol, mannitol, hexylene glycol, 1,3-butylene glycol, 1,2,6-hexanetriol, glycerol, ethoxylated glycerol, propoxylated glycerol, sodium 2-pyrrolidone-5-carboxylate, soluble collagen, gelatin, and mixtures thereof. Preferred moisturizing agents are glycerin, 1,3-butylene glycol, glucose, lactic acid, trimethylglycine, urea, or mixtures thereof; more preferably glycerin or 1,3-butylene glycol.

### D. Water

The compositions will comprise from 60% to 99.8% by weight, water, preferably from 80% to 99.5%.

In one embodiment, water further includes lower alkyl alcohols. Lower alkyl alcohols useful herein are C₁-C₆ alkyl monohydric alcohols; preferably C₂-C₃ alkyl alcohols. Preferred lower alkyl alcohols include ethyl alcohol, isopropyl alcohol, and mixtures thereof.

### E: Optional components

The transparent skin care composition of the present invention may further comprises a optional component. Herein, "optional component" means one or more compatible solid or liquid fillers, diluents, extenders and the like, which are commonly used in cosmetics as defined herein. The term "compatible" herein means that the components of the compositions of this invention are capable of being commingled with each other, in a manner such that there is no interaction which would substantially reduce the efficacy of the composition under ordinary use situations.

The optional component useful herein include a water-soluble polymeric thickening agent, a pH adjuster, and an active. The type of the optional component utilized in the present invention depends on the type of the product desired and may comprise several types of carriers including, but not limited to, oil-in-water or water-in-oil emulsion.

### 1) Water-Soluble Polymeric Thickening Agent

The optional component useful herein includes a water-soluble polymeric thickening agent. Preferably, the water-soluble polymeric thickening agent is present from 0.0001% to 0.15%, more preferably from 0.001% to 0.1% in the skin care composition.

It is believed that a higher level (*i*.*e*., greater than 0.15%) of the water-soluble polymeric thickening agent in the composition will result in an undesirably tacky feeling composition. It is also believed a lower concentration of the water soluble polymeric thickening agent (*i*.*e*., less than 0.0001%) may result in insufficient moisturization of the skin.

Water-soluble polymeric thickening agents useful herein include polysaccharides gums, mucopolysaccharides (*e.g*., hyaluronic acid, chondroitin sulfate), carboxylic acid polymers, crosslinked polyacrylate polymers, and mixtures thereof; preferably polysaccharides, gums, or mixtures thereof.

Extract materials which are derived from natural sources (*e.g*., Quince Seed) can be included as water-soluble polymeric thickening agent. Quince Seed is available from Taiyo Kagaku (Mie prefecture, Japan).

(i) Polysaccharide: A wide variety of polysaccharides can be used in the composition. Herein, "polysaccharides" refers to thickening agents containing a backbone of repeating sugar (*e.g*., carbohydrate) units. Examples of useful polysaccharides include those selected from the group consisting of cellulose, carboxymethyl hydroxyethylcellulose, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof; more preferably hydroxypropylcellulose.

In the above examples of useful polysaccharides, the hydroxy groups of the cellulose polymer are preferably hydroxyalkylated (preferably hydroxyethylate or hydroxypropylate), forming a hydroxyalkylated cellulose that is further modified with a straight or branched alkyl group of from 10 to 30 carbons through an ether linkage. Preferred polysaccharides are ethers of straight or branched alcohols of from 10 to 30 carbons with hydroxyalkylcelluloses.

Additional examples of useful polysaccharides include alkyl substituted cellulose. Examples of the alkyl groups useful herein include stearyl, isostearyl, lauryl, myristyl, cetyl, isocetyl, cocoyl (*e*.*g*., alkyl groups derived from the alcohols of coconut oil), palmityl, oleyl, linoleyl, linolenyl, ricinoleyl, behenyl, and mixtures thereof. Preferred among the alkyl hydroxyalkyl cellulose ethers herein is the material given the CTFA designation cetyl hydroxyethylcellulose, which is the ether of cetyl alcohol and hydroxyethylcellulose. This material is sold under the tradename Natrosol® CS Plus from Aqualon Corporation (Willmington, U.S.A.).

Other polysaccharides useful herein include scleroglucans containing a linear chain of (1 to less than 3) linked glucose units with a (1 to less than 6) linked glucose every three units. A commercially available example of this is Clearogel™ CS11 from Michel Mercier Products Inc. (Mountainside, NJ, U.S.A.).

(ii) Gum: Other water-soluble polymeric thickening agents which can be employed in the composition of the present invention include materials which are primarily derived from natural sources. Examples of such water soluble polymeric thickening agents include gums selected from the group consisting of acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, sodium hyaluroinate, hydroxypropyl chitosan, hydroxypropyl guar, karaya gum, kelp, locust bean gum, natto gum, potassium alginate, potassium carrageenan, propylene glycol alginate, sclerotium gum, sodium carboxymethyl dextran, sodium carrageenan, tragacanth gum, xanthan gum, and mixtures thereof.

Additional thickening gelling agents which are suitable herein as water-soluble polymeric thickening agents include those disclosed in U.S. Patent 4,387,107, Klein et al., issued June 7, 1983 and "Encyclopedia of Polymer and Thickeners for Cosmetics," R.Y. Lochhead and W. R. Fron, eds., Cosmetics & Toiletries, vol. 108, pp. 95-135 (May 1993).

### 2) pH Adjuster

The optional component also contain a pH adjuster. Herein, "pH adjuster" refers to any component which is employed to increase or decrease the overall pH of the composition to an optimum pH, thereby preventing unmet skin feeling such as skin irritation. An optimum pH is subject to the selection of preventing skin irritation. Preferably, the optimum pH is around 5.0 to about 7.0. Suitable pH adjusters herein include acetate, phosphate, citrate, triethanolamine and carbonate. A combination of the foregoing are often employed to adjust to a specific optimal pH for the composition. The total level by weight of total composition of the pH adjuster is from 0.01% to 5.0%, preferably, from 0.5% to 2.0%.

### 3) Actives

The optional component useful herein also contain actives. Examples of such actives include, a vitamin B₃ compound, an ascorbic acid compound, anti-oxidants and radical scavengers, anti-inflammatory agents, antimicrobial agents, sunscreens and sunblocks, and chelators. Other actives useful herein include vitamin A (e.g., retinoid which are commercially available from a number of sources, for example, Sigma Chemical Company (St. Louis, MO), and Boerhinger Mannheim (Indianapolis, IN) and described in U.S. Patent 4,677,120, Parish et al., issued Jun. 30, 1987; U.S. Patent 4,885,311, Parish et al., issued Dec. 5, 1989; U.S. Patent 5,049,584, Purcell et al., issued Sep. 17, 1991; U.S. Patent 5,124,356, Purcell et al., issued Jun. 23, 1992; and Reissue Patent 34,075, Purcell et al., issued Sep. 22, 1992); and vitamin K.

(i) Vitamin B₃ Compounds: The vitamin B₃ compound enhances the skin appearance benefits of the present invention especially, in regulating skin condition, including regulating signs of skin aging, more especially wrinkles lines and pores. The vitamin B₃ compound preferably present from 0.01% to 50%, more preferably from 0.1% to 10%, even more preferably from 0.5% to 10%, and still more preferably from 1% to 5%.

Herein. "vitamin B₃ compound" means a compound having the formula : 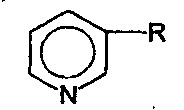 wherein R is -CONH₂ (*e*.*g*., niacinamide), -COOH (*e*.*g*., nicotinic acid) or -CH₂OH (*e*.*g*., nicotinyl alcohol); derivatives thereof; and salts of any of the foregoing.

Exemplary derivatives of the foregoing vitamin B₃ compounds include nicotinic acid esters, including non-vasodilating esters of nicotinic acid, nicotinyl amino acids, nicotinyl alcohol esters of carboxylic acids, nicotinic acid N-oxide and niacinamide N-oxide.

Suitable esters of nicotinic acid include nicotinic acid esters of from 1 to 22 carbons, preferably 1 to 16 carbons, more preferably alcohols from 1 to 6 carbons. The alcohols are suitably straight-chain or branched chain, cyclic or acyclic, saturated or unsaturated (including aromatic), and substituted or unsubstituted. The esters are preferably non-vasodilating. As used herein, "non-vasodilating" means that the ester does not commonly yield a visible flushing response after application to the skin in the subject compositions (the majority of the general population would not experience a visible flushing response, although such compounds may cause vasodilation not visible to the naked eye, *i*.*e*., the ester is non-rubifacient). Non-vasodilating esters of nicotinic acid include tocopherol nicotinate and inositol hexanicotinate; tocopherol nicotinate is preferred.

Other derivatives of the vitamin B₃ compound are derivatives of niacinamide resulting from substitution of one or more of the amide group hydrogens. Examples of derivatives of niancinamide useful herein include nicotinyl amino acids, derived, for example, from the reaction of an activated nicotinic acid compound (*e*.*g*., nicotinic acid azide or nicotinyl chloride) with an amino acid, and nicotinyl alcohol esters of organic carboxylic acids (*e*.*g*., 1 to about 18 carbons). Specific examples of such derivatives include nicotinuric acid (C₈H₈N₂O₃) and nicotinyl hydroxamic acid (C₆H₆N₂O₂), which have the following chemical structures:
nicotinuric acid: 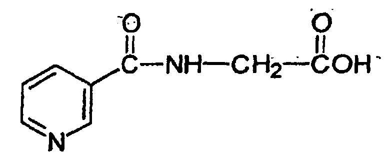 nicotinyl hydroxamic acid:

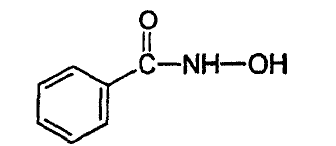

Exemplary nicotinyl alcohol esters include nicotinyl alcohol esters of the carboxylic acids salicylic acid, acetic acid, glycolic acid, palmitic acid and the like. Other non-limiting examples of vitamin B₃ compounds useful herein are 2-chloronicotinamide, 6-aminonicotinamide, 6-methylnicotinamide, n-methyl-nicotinamide, n,n-diethylnicotinamide, n-(hydroxymethyl)-nicotinamide, quinolinic acid imide, nicotinanilide; n-benzylnicotinamide, n-ethylnicotinamide, nifenazone, nicotinaldehyde, isonicotinic acid, methyl isonicotinic acid, thionicotinamide, nialamide, 1-(3-pyridylmethyl) urea, 2-mercaptonicotinic acid, nicomol, and niaprazine.

Examples of the above vitamin B₃ compounds are well known in the art and are commercially available from a number of sources, *e.g.*, the Sigma Chemical Company (St. Louis, MO); ICN Biomedicals, Inc. (Irvin, CA) and Aldrich Chemical Company (Milwaukee, WI).

One or more vitamin B₃ compounds may be used herein. Preferred vitamin B₃ compounds are niacinamide and tocopherol nicotinate. Niacinamide is more preferred.

When used, salts derivatives, and salt derivatives of niacinamide are preferably those having substantially the same efficacy as niacinamide in the methods of regulating skin condition described herein.

Salts of the vitamin B₃ compound are also useful herein. Examples of salts of the vitamin B₃ compound useful herein include organic or inorganic salts, such as inorganic salts with anionic inorganic species (*e.g.*, chloride, bromide, iodide, carbonate, preferably chloride), and organic carboxylic acid salts (including mono-, di- and tri- C₁₋₁₈ carboxylic acid salts, *e*.*g*., acetate, salicylate, glycolate, lactate, malate, citrate, preferably monocarboxylic acid salts such as acetate). These and other salts of the vitamin B₃ compound can be readily prepared by the skilled artisan, for example, as described by W. Wenner, "The Reaction of L-Ascorbic and D-losascorbic Acid with Nicotinic Acid and Its Amide", J. Organic Chemistry, Vol. 14, 22-26 (1949). Wenner describes the synthesis of the ascorbic acid salt of niacinamide.

In a preferred embodiment, the ring nitrogen of the vitamin B₃ compound is substantially chemically free (*e*.*g*., unbound and/or unhindered), or after delivery to the skin becomes substantially chemically free ("chemically free" is hereinafter alternatively referred to as "uncomplexed"). More preferably, the vitamin B₃ compound is essentially uncomplexed. Therefore, if the composition contains the vitamin B₃ compound in a salt or otherwise complexed form, such complex is preferably substantially reversible, more preferably essentially reversible, upon delivery of the composition to the skin. For example, such complex should be substantially reversible at a pH of from 5.0 to 6.0. Such reversibility can be readily determined by one having ordinary skill in the art.

More preferably the vitamin B₃ compound is substantially uncomplexed in the composition prior to delivery to the skin. Exemplary approaches to minimizing or preventing the formation of undesirable complexes include omission of materials which form substantially irreversible or other complexes with the vitamin B₃ compound, pH adjustment, ionic strength adjustment, the use of surfactants, and formulating wherein the vitamin B₃ compound and materials which complex therewith are in different phases. Such approaches are well within the level of ordinary skill in the art.

Thus, in a preferred embodiment, the vitamin B₃ compound contains a limited amount of the salt form and is more preferably substantially free of salts of a vitamin B₃ compound. Preferably the vitamin B₃ compound contains less than 50% of such salt, and is more preferably essentially free of the salt form. The vitamin B₃ compound in the compositions hereof having a pH of from 4 to 7 typically contain less than 50% of the salt.

The vitamin B₃ compound may be included as the substantially pure material, or as an extract obtained by suitable physical and/or chemical isolation from natural (*e*.*g*., plant) sources. The vitamin B₃ compound is preferably substantially pure, more preferably essentially pure.

(ii) Ascorbic Acid Compound: The ascorbic acid compound may be included as a substantially pure material, for example, which may be an extract obtained by suitable physical and/or chemical isolation from natural (*e*.*g*., plant) sources.

Preferably, the ascorbic acid compound useful herein is an ascorbic acid salt or derivative thereof, such as the non-toxic alkali metal, alkaline earth metal and ammonium salts commonly known by those skilled in the art including, but not limited to, the sodium, potassium, lithium, calcium, magnesium, barium, ammonium and protamine salts which are prepared by methods well known in the art. More preferably, the ascorbic acid salt useful herein is a metal ascorbate.

Examples of other preferred ascorbic acid salts include monovalent metal salts (*e*.*g*., sodium ascorbate, potassium ascorbate), divalent metal salts (*e*.*g*., magnesium ascorbate, calcium ascorbate) and trivelent metal salts (e.g., aluminum ascorbate) of ascorbic acid.

Preferably, the ascorbic acid salt useful herein is a water soluble ascorbyl ester. Exemplary water soluble salt derivatives include, but are not limited to, L-ascorbyl phosphate ester salts such as sodium L-ascorbyl phosphate, potassium L-ascorbyl phosphate, magnesium L-ascorbyl phosphate, calcium L-ascorbyl phosphate, aluminum L-ascorbyl phosphate. L-ascorbyl sulfate ester salts can also be used. Examples are sodium L-ascorbyl sulfate, potassium L-ascorbyl sulfate, magnesium L-ascorbyl sulfate, calcium L-ascorbyl sulfate and aluminum L-ascorbyl sulfate.

(iii) Anti-Oxidants and Radical Scavengers: Anti-oxidants and radical scavengers are especially useful for providing protection against UV radiation which can cause increased scaling or texture changes in the stratum corneum and against other environmental agents which can cause skin damage.

Anti-oxidants and radical scavengers such as tocopherol (vitamin E), tocopherol sorbate, tocopherol acetate, other esters of tocopherol, butylated hydroxy benzoic acids and their salts, 6-hydroxy-2,5,7,8-tetramethylchroman-2-carboxylic acid (commercially available under the tradename Trolox®), gallic acid and its alkyl esters, especially propyl gallate, uric acid and its salts and alkyl esters, sorbic acid and its salts, amines (*i*.*e*., N,N-diethylhydroxylamine, aminoguanidine), sulfhydryl compounds (*i*.*e*., glutathione), dihydroxy fumaric acid and its salts, lycine pidolate, arginine pilolate, nordihydroguaiaretic acid, bioflavonoids, lysine, methionine, proline, superoxide dismutase, silymarin, tea extracts, grape skin/seed extracts, melanin, and rosemary extracts may be used. Preferred anti-oxidants/radical scavengers are selected from tocopherol sorbate and other esters of tocopherol, more preferably tocopherol sorbate. For example, the use of tocopherol sorbate in topical compositions and applicable to the present invention is described in U.S. Patent 4,847,071, Bissett et al, issued July 11, 1989.

(iv) Anti-Inflammatory Agents: Anti-inflammatory agents enhance the skin appearance benefits, by for example, contribution of uniformity and acceptable skin tone and/or color.

Preferably, the anti-inflammatory agent includes a steroidal anti-inflammatory agent and an non-steroidal anti-inflammatory agent. Preferred steroidal anti-inflammatory for use is hydrocortisone.

The variety of compounds encompassed by this group are well-known to those skilled in the art. For detailed disclosure of the chemical structure, synthesis, side effects, etc. of non-steroidal anti-inflammatory agents, reference may be had to standard texts, including Anti-inflammatory and Anti-Rheumatic Drugs, K. D. Rainsford, Vol. I-III, CRC Press, Boca Raton, (1985), and Anti-inflammatory Agents, Chemistry and Pharmacology, 1, R. A. Scherrer, et al., Academic Press, New York (1974).

So-called "natural" anti-inflammatory agents are also useful. Such agents may suitably be obtained as an extract by suitable physical and/or chemical isolation from natural sources (*i*.*e*., plants, fungi, by-products of microorganisms). For example, alpha bisabolol, aloe vera, Manjistha (extracted from plants in the genus Rubia, particularly Rubia Cordifolia), and Guggal (extracted from plants in the genus Commiphora, particularly Commiphora Mukul), kola extract, chamomile, and sea whip extract, may be used.

Additional anti-inflammatory agents useful herein include compounds of the licorice (the plant genus/species Glycyrrhiza glabra) family, including glycyrrhetic acid, glycyrrhizic acid, and derivatives thereof (*e*.*g*., salts and esters). Suitable salts of the foregoing compounds include metal and ammonium salts. Suitable esters include C₂₋₂₄ saturated or unsaturated esters of the acids, preferably C₁₀₋₂₄, more preferably C₁₆₋₂₄.

(v) Antimicrobial Agent: As used, "antimicrobial agents" means a compound capable of destroying microbes, preventing the development of microbes or preventing the pathogenic action of microbes. Antimicrobal agents are useful, for example, in controlling acne. Preferred antimicrobial agents useful in the present invention are benzoyl peroxide, erythromycin, tetracycline, clindamycin, azelaic acid, sulfur resorcinol phenoxyethanol, and Irgasan™ DP 300 (Ciba Geigy Corp., U.S.A.). A safe and effective amount of an antimicrobial agent may be added to compositions of the present invention, preferably from 0.001% to 10%, more preferably from 0.01% to 5%, still more preferably from 0.05% to 2%.

(vi) Sunscreens and Sunblocks: Sunscreens and sunblocks generally prevent excessive scaling and texture changes of the stratum corneum by exposure of ultraviolet light and may be added to the composition of the present invention. Suitable sunscreens and sunblocks may be organic or inorganic.

A wide variety of conventional sunscreens and sunblocks are suitable for use herein. See, U.S. Patent 5,087,445, Haffey et al, issued February 11, 1992; U.S. Patent 5,073,372, Turner et al, issued December 17, 1991; U.S. Patent 5,073,371, Turner et al., issued December 17, 1991; and Segarin, et al, at Chapter VIII, pages 189 et seq., of Cosmetics Science and Technology (1972), which discloses numerous suitable sunscreens and sunblocks. Preferred among those sunscreens and sunblocks which are useful in the compositions are those selected from 2-ethylhexyl-p-methoxycinnamate (commercially available as PARSOL MCX), butylmethoxydibenzoyl-methane, 2-hydroxy-4-methoxybenzophenone, 2-phenylbenzimidazole-5-sulfonic acid, octyldimethyl-p-aminobenzoic acid, octocrylene, 2-ethylhexyl N,N-dimethyl-p-aminobenzoate, p-aminobenzoic acid, 2-phenylbenzimidazole-5-sulfonic acid, octocrylene, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, titanium dioxide, zinc oxide, silica, iron oxide, Eusolex™ 6300, Octocrylene, Parsol 1789, and mixtures thereof.

Also particularly useful in the compositions are sunscreens and sunblocks such as those disclosed in U.S. Patent 4,937,370, Sabatelli, issued June 26, 1990, and U.S. Patent 4,999,186, Sabatelli, issued March 12, 1991. The sunscreens and sunblocks disclosed therein have, in a single molecular, two distinct chromophore moieties which exhibit different ultraviolet radiation absorption spectra. One of the chromophore moieties absorbs predominantly in the UVB radiation range and the other absorbs strongly in the UVA radiation range. These sunscreens and sunblocks provide higher efficacy, broader UV absorption, lower skin penetration and longer lasting efficacy relative to conventional sunscreens and sunblocks.

Exact amounts will vary depending upon the sunscreen chosen and the desired Sun Protection Factor (SPF). SPF is a commonly used measure of photoprotection of a sunscreen against erythema. See Federal Register, Vol. 43, No. 166. pp. 38206-38269, August 25, 1978.

A sunscreen or sunblock herein may also be added to improve the skin, particularly to enhance their resistance to being washed off by water, or rubbed off. Preferred sunscreens and sunblocks which will provide this benefit are a copolymer of ethylene and acrylic acid. Compositions comprising this copolymer are disclosed in U.S. Patent 4,663,157, Brock, issued May 5, 1987.

(vii) Chelators: As used herein, "chelator" refers to a compound that reacts for removing a metal ion from a system by forming a complex so that the metal ion cannot readily participate in or catalyze chemical reactions. The inclusion of a chelator is especially useful for providing protection against UV radiation which can contribute to excessive scaling or skin texture changes and against other environmental agents which can cause skin damage.

Exemplary chelators that are useful herein are disclosed in U.S. Patent 5,487,884, Bissett et al, issued January 30, 1996; PCT application 91/16035 and 91/16034, Bush et al, published October 31, 1995. Preferred chelators are furildioxime and derivatives thereof.

### 4) Other components

In addition to the above described components, the composition of the present invention may further include preservatives and preservative enhancers such as water-soluble or solubilizable preservatives including Germall 115, methyl, ethyl, propyl and butyl esters of hydroxybenzoic acid, benzyl alcohol, EDTA, Bronopol (2-bromo-2-nitropropane-1,3-diol) and phenoxypropanol; other skin lightening/evenness agents including kojic acid and arbutin; WO95/23780, Kvalnes et al, published September 8, 1995; skin-conditioning agents; skin penetration enhancing agents; skin protectants; skin soothing agents; skin healing agents; ultraviolet light absorbers or scattering agents; sequestrants; anti-acne agents; anti-androgens; depilation agents; keratolytic agents/desquamation agents/ exfoliants such as salicylic acid; panthenol moisturizer such as D-panthenol; soluble or colloidally-soluble moisturizing agents such as hyaluronic acid and starch-grafted sodium polyacrylates such as Sanwet™ IM-1000, IM-1500 and IM-2500 available from Celanese Superabsorbent Materials, Portsmith, VA, USA and described in US Patent 4,076,663; proteins and polypeptides and derivatives thereof; organic hydroxy acids; drug astringents; external analgesics; film formers; absorbents including oil absorbents such as clays and polymeric absorbents; abrasives; anticaking agents; antifoaming agents; binders; biological additives; bulking agents; coloring agents; perfumes, essential oils, and solubilizers thereof; natural extracts; compounds which stimulate collagen production.

### E. Method for Making Composition

The compositions of the present invention are generally prepared by any method conventionally used for providing skin care compositions, particularly for skin lotions, that are known in the art. Such methods typically involve mixing of the ingredients in one or more steps to a relatively uniform state, with or without heating, cooling, and the like. Typical methods are described in, for example are described in Harry's Cosmeticology, 7th Ed., Harry & Wilkinson (Hill Publishers, London 1982).

### EXAMPLES

The examples I and IV-V further describe and demonstrate embodiments within the scope of the present invention. The examples are given solely for the purpose of illustration and are not to be construed as limitations of the present invention. Examples II and III do not fall within the scope of the invention. Where applicable, ingredients are identified by chemical or CTFA name, or otherwise defined below.

The compositions shown below can be prepared by any conventional method known in the art. Suitable methods and formulations are as follows:

| | | | | | | |
|---|---|---|---|---|---|---|
| (unit weight %) | | | | | | |

| | | I | II | III | IV | V |
|---|---|---|---|---|---|---|
| phase A | POE-40 sorbit tetraoleate | 0.2 | 0.6 | 0.8 | - | - |
| | POE-60 sorbit tetrastearate | - | - | - | 0.2 | 0.2 |
| | POE-20 hydrogenated caster oil | 0.4 | - | 0.2 | 0.2 | 0.4 |
| | Na POE-5 cetyl phosphate | 0.4 | 0.4 | - | 0.6 | 0.4 |
| | Fatty acid ester of sugar 1 | 0.5 | 0.5 | 0.5 | - | - |
| | squalane | - | - | - | 0.5 | - |
| | isostearyl isostearate | - | - | - | - | 0.5 |
| phase B | glycerin | 2.0 | 2.0 | 2.0 | 2.0 | 2.0 |
| | preservative | 0.1 | 0.1 | 0.1 | 0.1 | 0.1 |
| phase C | de-ionized water | up to 100 | | | | |

Fatty acid ester of sugar ¹: A C1-C30 monoester or polyester of sugars and one or more carboxylic acid moieties as described herein, preferably a sucrose polyester in which the degree of esterification is 7-8, and in which the fatty acid moieties are C18 mono- and/or di-unsaturated and behenic, in a molar ratio of unsaturates:behenic of 1:7 to 3:5, more preferably the octaester of sucrose in which there are about 7 behenic fatty acid moieties and about 1 oleic acid moiety in the molecule, *e.g.*, sucrose ester of cottonseed oil fatty acids,

The compositions above described are suitably made as follows:
1. Mix phase A ingredients using propeller type and a suitably size vessel mixer and heat to 70-75°C, if the ingredients are insolid form.
2. Add phase B ingredients to phase A mixture and mix at 70-75°C until the phase B ingredients melt completely.
3. Add water.

The embodiments disclosed and represented by the previous examples have many advantages. For example, the composition comprising specic surfactant combination and oil compounds herein provide improved moisturization of the skin without imparting a greasy feeling and/or provide good spreadability during use.

## Claims

1. A transparent skin care composition comprising:
(a) a surfactant combination comprising i), ii) and iii):
i) polyoxyethylene sorbit tetralkyl ester;
ii) polyoxyethylene castor oil ester and/or polyoxyethylene hydrogenated castor oil ester; and
iii) polyoxyethylene alkyl phosphate or salts
(b) an oil compound
(c) a polyhydric alcohol; and
(d) water.
wherein the oil compound is substantially solubilized in the transparent skin care composition.

2. The transparent skin care composition of Claim 1, wherein the ratio of the surfactant to the oil compound is less than 1.0.

3. The transparent skin care composition of Claim 2, wherein the ratio of polyoxyethylene sorbit tetraalkyl ester to polyoxyethylene castor oil ester and/or polyoxyethylene hydrogenated castor oil ester is from 4 : 1 to 2 : 3.

4. The transparent skin care composition of Claim 2, wherein the ratio of polyoxyethylene alkyl phosphate or salts to polyoxyethylene castor oil ester and/or polyoxyethylene hydrogenated castor oil ester is from 4 : 1 to 2 : 3.

5. The transparent skin care composition of Claim 2, wherein the ratio of polyoxyethylene sorbit tetraalkyl ester to polyoxyethylene alkyl phosphate or salts is from 3 : 2 to 2 : 3.

6. The transparent skin care composition of Claim 1, wherein the oil compound is selected from the group consisting of mineral oil, petrolatum, C₇₋₄₀ straight and branched hydrocarbons, C₁₋₃₀ alcohol esters of C₁₋₃₀ carboxylic acids and of C₂₋₃₀ dicarboxylic acids, vegetable oils and hydrogenated vegetable oils, animal fats and oils, and C₄₋₂₀ alkyl ethers of polypropylene glycols, C₁₋₂₀ carboxylic acid esters of polypropylene glycols, di-C₈₋₃₀ alkyl ethers, essential oils, and a liquid polyol carboxylic acid ester having a polyol moiety and at least 4 carboxylic acid moieties ester.

7. The transparent skin care composition of Claim 1, wherein the composition further comprising an optional component selected from the group consisting of a water-soluble polymeric thickening agent, a pH adjuster, and an active.

8. The transparent skin care composition of Claim 1 comprising:
(a) from 0.001 to 5.0% of the surfactant combination
(b) from 0.001 to 5.0% of the oil compound;
(c) from 0.01 to 10% of the polyhydric alcohol; and
(d) from 60 to 99.8% of water;
wherein the oil compound is substantially solubilized in the transparent skin care composition.

## Patentansprüche

1. Transparente Hautpflegezusammensetzung, umfassend:
(a) eine Tensidkombination, umfassend i), ii) und iii):
i) Polyoxyethylensorbittetraalkylester;
ii) Polyoxyethlyenkastorölester und/oder hydrierter Polyoxyethylenkastorölester; und
iii) Polyoxyethylenalkylphosphat oder Salze
(b) eine Ölverbindung
(c) einen mehrwertigen Alkohol; und
(d) Wasser,
wobei die Ölverbindung größtenteils solubilisiert in der transparenten Hautpflegezusammensetzung vorliegt.

2. Transparente Hautpflegezusammensetzung nach Anspruch 1, wobei das Verhältnis des Tensides zu der Ölverbindung weniger als 1,0 beträgt.

3. Transparente Hautpflegezusammensetzung nach Anspruch 2, wobei das Verhältnis von Polyoxyethylensorbittetraalkylester zu Polyoxyethylenkastorölester und/oder hydriertem Polyoxyethylenkastorölester von 4:1 bis 2.3 reicht.

4. Transparente Hautpflegezusammensetzung nach Anspruch 2, wobei das Verhältnis von Polyoxyethylenalkylphosphat oder Salzen zu Polyoxyethlyenkastorölester und/oder hydriertem Polyoxyethylenkastorölester von 4:1 bis 2:3 reicht.

5. Transparente Hautpflegezusammensetzung nach Anspruch 2, wobei das Verhältnis von Polyoxyethylensorbittetraalkylester zu Polyoxyethylenalkylphosphat oder Salzen von 3:2 bis 2:3 reicht.

6. Transparente Hautpflegezusammensetzung nach Anspruch 1, wobei die Ölverbindung ausgewählt ist aus der Gruppe, bestehend aus Mineralöl, Petrolatum, geradkettigen und verzweigten C₇₋₄₀-Kohlenwasserstoffen, C₁₋₃₀-Alkoholestern von C₁₋₃₀-Carbonsäuren und von C₂₋₃₀-Dicarbonsäuren, Pflanzenölen und hydrierten Pflanzenölen, tierischen Fetten und Ölen, und C₄₋₂₀-Alkylethern von Polypropylenglykolen, C₁₋₂₀-Carbonsäureestern von Polypropylenglykolen, Di-C₈₋₃₀-Alkylethern, ätherischen Ölen, und einem flüssigen Polyolcarbonsäureester mit einer Polyolestereinheit und mindestens vier Carbonsäureestereinheiten.

7. Transparente Hautpflegezusammensetzung nach Anspruch 1, wobei die Zusammensetzung weiterhin eine wahlweise Komponente umfasst, ausgewählt aus der Gruppe, bestehend aus einem wasserlöslichen polymeren Verdickungsmittel, einem pH-Einstellmittel und einem Wirkstoff.

8. Transparente Hautpflegezusammensetzung nach Anspruch 1, umfassend:
(a) 0,001 bis 5,0% der Tensidkombination
(b) 0,001 bis 5,0% der Ölverbindung;
(c) 0,01 bis 10% des mehrwertigen Alkohols; und
(d) 60 bis 99,8% Wasser;
wobei die Ölverbindung größtenteils solubilisiert in der transparenten Hautpflegezusammensetzung vorliegt.

## Revendications

1. Composition transparente de soins pour la peau comprenant :
(a) une combinaison d'agents tensioactifs comprenant :
i) un tétraalkylester de sorbitol et de polyoxyéthylène;
ii) un ester d'huile de ricin et de polyoxyéthylène et/ou un ester d'huile de ricin hydrogénée et de polyoxyéthylène; et
iii) un phosphate d'alkyle et de polyoxyéthylène ou ses sels ;
(b) un composé d'huile;
(c) un alcool polyvalent; et
(d) de l'eau;
dans laquelle le composé d'huile est essentiellement solubilisé dans la composition transparente de soins pour la peau.

2. Composition transparente de soins pour la peau selon la revendication 1, dans laquelle le rapport de l'agent tensioactif au composé d'huile est inférieur à 1,0.

3. Composition transparente de soins pour la peau selon la revendication 2, dans laquelle le rapport du tétraalkylester de sorbitol et de polyoxyéthylène à l'ester d'huile de ricin et de polyoxyéthylène et/ou à l'ester d'huile de ricin hydrogénée et de polyoxyéthylène est de 4:1 à 2:3.

4. Composition transparente de soins pour la peau selon la revendication 2, dans laquelle le rapport du phosphate d'alkyle et de polyoxyéthylène ou de ses sels à l'ester d'huile de ricin et de polyoxyéthylène et/ou à l'ester d'huile de ricin hydrogénée et de polyoxyéthylène est de 4:1 à 2:3.

5. Composition transparente de soins pour la peau selon la revendication 2, dans laquelle le rapport du tétraalkylester de sorbitol et de polyoxyéthylène au phosphate d'alkyle et de polyoxyéthylène ou à ses sels est de 3:2 à 2:3.

6. Composition transparente de soins pour la peau selon la revendication 1, dans laquelle le composé d'huile est choisi dans le groupe constitué d'une huile minérale, de vaseline, des hydrocarbures à chaînes droites et ramifiées en C₇₋₄₀, des esters d'alcools en C₁₋₃₀ et d'acides carboxyliques en C₁₋₃₀ et d'acides dicarboxyliques en C₂₋₃₀, des huiles végétales et des huiles végétales hydrogénées, des graisses et des huiles animales et des alkyléthers en C₄₋₂₀ de polypropylèneglycols, des esters d'acides carboxyliques en C₁₋₂₀ et de polypropylèneglycols, des éthers de di-alkyle en C₈₋₃₀, des huiles essentielles et d'un ester d'acide carboxylique et de polyol liquide ayant un radical polyol et au moins 4 radicaux d'acide carboxylique.

7. Composition transparente de soins pour la peau selon la revendication 1, dans laquelle la composition comprenant en outre un composant facultatif choisi dans le groupe constitué d'un agent épaississant polymère soluble dans l'eau, d'un régulateur de pH et d'un ingrédient actif.

8. Composition transparente de soins pour la peau selon la revendication 1, comprenant :
a) 0,001% à 5,0% de la combinaison d'agents tensioactifs;
b) 0,001% à 5,0% du composé d'huile;
c) 0,01% à 10% de l'alcool polyvalent; et
d) 60% à 99,8% d'eau;
dans laquelle le composé d'huile est essentiellement solubilisé dans la composition transparente de soins pour la peau.
